(19) Europäisches Patentamt
European Patent Office
Office européen des brevets

(11) **EP 0 815 253 B1**

(12) **EUROPÄISCHE PATENTSCHRIFT**

(45) Veröffentlichungstag und Bekanntmachung des Hinweises auf die Patenterteilung:
**20.06.2001 Patentblatt 2001/25**

(21) Anmeldenummer: **96907302.2**

(22) Anmeldetag: **21.03.1996**

(51) Int Cl.$^7$: **C12P 1/00**, A61K 35/12
// (C12P1/00, C12R1:91)

(86) Internationale Anmeldenummer:
**PCT/DE96/00550**

(87) Internationale Veröffentlichungsnummer:
**WO 96/30537 (03.10.1996 Gazette 1996/44)**

(54) **MULTIFAKTORIELLES ABWEHR-MODULATORENGEMISCH, VERFAHREN ZU SEINER HERSTELLUNG UND DIESE MODULATOREN ENTHALTENDE ARZNEIMITTEL**

MULTI-FACTOR IMMUNITY MODULATOR MIXTURE, PROCESS FOR ITS PRODUCTION AND MEDICAMENTS CONTAINING SAID MODULATORS

MELANGE DE MODULATEURS D'IMMUNITE MULTIFACTORIEL, SON PROCEDE DE FABRICATION ET MEDICAMENTS RENFERMANT CES MODULATEURS

(84) Benannte Vertragsstaaten:
**AT BE CH DK ES FR GB GR IE IT LI NL PT SE**

(30) Priorität: **24.03.1995 DE 19512227**

(43) Veröffentlichungstag der Anmeldung:
**07.01.1998 Patentblatt 1998/02**

(73) Patentinhaber: **Privates Institut Bioserv GmbH**
**18059 Rostock (DE)**

(72) Erfinder:
• **HEINRICH, Hans-Werner**
**D-17498 Riemserort (DE)**
• **MEYER, Udo**
**D-18239 Hastorf (DE)**
• **BANGE, Volker**
**D-18069 Rostock (DE)**
• **TECH, Egon**
**D-18334 Bad Sülze (DE)**

(74) Vertreter: **Baumbach, Friedrich et al**
**BioTeZ Berlin-Buch GmbH**
**Patentstelle**
**Robert-Rössle-Strasse 10**
**13125 Berlin (DE)**

(56) Entgegenhaltungen:
DE-A- 3 504 940

• **DE-G-9218127.9(P.SOLISCH ET AL.) 16-12-93 XP002009282 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein multifaktorielles Abwehr-Modulatorengemisch, insbesondere multipotente Modulatoren des Abwehrsystems, Verfahren zu seiner Herstellung und diese Modulatoren enthaltende Arzneimittel.

Die Mechanismen zur Erhaltung der Integrität des Organismus entstanden parallel mit der Herausbildung der Lebensformen. Ohne Abwehr schädlicher Umwelteinflüsse wäre die Entwicklung des Lebens nicht möglich gewesen.

Bestimmte Reaktionsmöglichkeiten der Einzeller auf schädigende Umwelteinflüsse durch die Synthese von "Stressproteine" zu reagieren blieben im Verlaufe der Phylogenese erhalten und wurden ergänzt durch die spezifischen Abwehrleistungen von spezialisierten Zellen und Geweben, die einer fein abgestimmten multifaktoriellen Regulierung unterliegen. Als extrazelluläre Botenstoffe wurden eine Reihe von Proteinen erkannt, die als Lymphokine bzw. Cytokine, wie Interferone, Interleukine und Wachtumsfaktoren, von bestimmten Zellen freigesetzt, bei anderen eine Vielzahl von Reaktionen, teils gegensätzlicher Art auslösen.

In Kultur gehaltene somatische Zellen produzieren noch Substanzen, die eine Signalübertragungsfunktion ausüben. In der Zellzucht werden seit Jahrzehnten für die Anzüchtung empfindlicher bzw. sehr weniger Zellen "konditionierte" Medien verwendet. Das ist eine Mischung von frischem Medium mit Überständen von Zellkulturen. Auch der Einsatz von "Feeder-Zellen" bei der Etablierung von Hybridom-Zellinien erfüllt den gleichen Zweck, die Bereitstellung von biologisch aktiven Substanzen, die die Entwicklung anderer Zellen positiv befördern.

Zur Unterstützung der Heilkraft werden seit Jahrhunderten die verschiedensten Substanzen eingesetzt, die, von Pflanzen, Tieren oder Mikroorganismen gewonnen, den erkrankten Organismus direkt unterstützen, bzw. die krankmachenden Agentien schädigen oder zerstören.

Oft handelt es sich um Stoffgemische, die Ihre Wirksamkeit nur eben in der jeweiligen Mischung entfalten. Der Wirkungsmechanismus wird meist nur durch das Aufzählen von erwünschten und Nebenwirkungen beschrieben. Für die molekularbiologischen Abläufe und Wechselwirkungen existieren meist nur Hypothesen bzw. sehr begrenzte Einsichten.

Aber auch "Design-Pharmaka" entwickeln nicht nur die beabsichtigten Wirkungen, sondern setzten häufig Regelkreise in Gang, die im Interesse des therapeutischen Effektes in Kauf genommen werden.

Die funktionelle Grundlage für die Erhöhung der Abwehrlage sind komplex und beruhen hauptsächlich auf der Steigerung der Phagozytoseleistung, der Stimulierung humoraler Abwehrfaktoren, insbesondere des Opsonin-Properdin-Komplement-Systems, auf der Aktivierung des lymphopoetischen Systems und der Freisetzung von Cytokinen. Je nach der Art der Stimulierung können die vorherrschenden Abwehrleistungen unterschiedlich sein.

Diese Steigerung der Abwehrlage wird auf natürliche Weise im Verlauf eines Infektionsgeschehens oder künstlich, d.h. medikamentös, erworben. Das erfolgt sowohl systemisch als auch lokal über die Schleimhäute des Respirations-, Digestions- und Urogenitaltraktes.

Eine solche erhöhte Abwehrlage kann mit unterschiedlichen biologischen und chemischen Mitteln erzielt werden. Die Anwendung von dieser Präparaten ist immer dann die Methode der Wahl, wenn die klassischen Verfahren zur Prophylaxe und Therapie von Infektionskrankheiten und Tumorerkrankungen versagen. Hauptsächlich handelt es sich dabei um die Bekämpfung von infektiösen Faktorenkrankheiten, Mischinfektionen, infektiösem Hospitalismus, chronischen Verlaufsformen von Infektionskrankheiten, persistierenden Infektionen und ihren immunpathogenen Folgen, Chemotherapie-resistenten Infektionskrankheiten, Autoimmunerkrankungen, Therapieversagen bei medikamentell bzw. andersweitig immunsupprimierten Patienten.

Die Induktion einer erhöhten Abwehrlage kann Neuerkrankungen der vorstehend erwähnten Art in ihrer Entstehung und Ausbreitung verhindern und bestehende Erkrankungen heilen oder in ihrer Ausbreitung verzögern. Dadurch können kurzfristig Infektionen und Krankheiten verschiedenster Art für den Patienten günstig beeinflußt werden.

Natürlicherweise reagiert der Organismus sofort auf eine Infektion. Die Qualität und Quantität der Sofortreaktion hängt ab von der Art des Antigens und dem Ort des Eindringens. Prinzipiell gleiche Reaktionen erfolgen bei Verabreichung von Impfstoffen und anderen körperfremden Substanzen. Eine spezifische Abwehr, meßbar z.B. durch den Nachweis spezifischer Antikörper, ist erst nach einigen Tagen wirksam.

Die unspezifische Sofortreaktion entwickelt sich innerhalb weniger Stunden und hält unterschiedlich lange an. Im Gegensatz zur spezifischen Immunantwort verbleibt nach dem Absinken der erhöhten Reaktionslage nichts im Organismus zurück (spezifische B- und/oder T-Zellen, spezifische Antikörper), was auf das auslösende Moment schließen läßt.

Als solche unspezifische Induktoren eignen sich unterschiedlichste Präparate. Experimentelle bzw. klinische Erfahrungen liegen vor mit

1. Interferon-Inducern
wie natürliche und synthetische Nukleinsäuren, synthetische Polyanionen

2. Lymphozytenstimulantien

wie unspezifische Mitogene (z.B. Concanavalin A, Phythämagglutinin), Thymosin, Lymphokine

3. Synthetische Substanzen
wie Levamisol, Tiloron, Isoprinosin

4. Impfstoffen
gegen Influenza, Parainfluenza, Newcastle Disease, Infektiöse Bovine Rhinotracheitis

5. Antigenaufbereitungen
wie Extrakte aus Blut oder Organen (z.B. Thymus, Milz, Plazenta), pyrogenfreie Bakterien- oder Pilzextrakte (z. B. BCG, Brucellen, Salmonellen, Escherichia coli, Mycobacterium bovis, Corynebacterium parvum), bakterielle Ribosomen, Pflanzenextrakte, Dextrane, Lipide, Protein-Spaltprodukte

6. Aufbereitungen aus infizierten Zellkulturen
wie PIND-AVI

7. Aufbereitungen partikulärer Bestandteile aus Zellkulturen
wie Partikuläre Biokomplexe

8. Aufbereitungen aus Blutlymphozyten
wie LeukoNorm

Fast allen dieser Stoffe ist gemeinsam, daß sie biologischen Ursprungs sind und häufig als Antigene wirken. Die einsetzende Immunreaktion verändert die Wirksamkeit einer wiederholten Applikation. Meist sind sie nur schwer standardisierbar und z.T. mit solchen Nebenwirkungen verbunden, daß eine praktischen Anwendung ausgeschlossen ist. Die mögliche Weiterverbreitung von noch unbekannten bzw. nicht erkannten Krankheiten verbietet den breiten Einsatz von Blut-oder Organextrakten, es sei denn, eine Erregerübertragung kann sicher ausgeschlossen werden.
Die Wirkung der bekannten Induktoren der unspezifischen Abwehr setzt das Vorhandensein funktionstüchtiger Abwehrzellen (Phagozyten und Lymphozyten) voraus. Sie läuft als Mehrstufenreaktion ab. In der Initialphase erfolgt die Stimulierung von Induktorzellen (Makrophagen, Granulozyten, bestimte B- und T-Zell-Subpopulationen u.a.), die zur Produktion und/oder Freisetzung von Botenstoffen veranlaßt werden. Je nach Art dieser Lympho- oder Cytokine werden verschiedene Zellgruppen angeregt (Makrophagen, Granulozyten, bestimmte B- und T-Zell-Subpopulationen u.a.). Diese Reaktionen sind vorerst unspezifisch, beinhalten aber bereits Elemente der Immunantwort. Einmal in Gang gesetzt, richten sie sich allgemein gegen körperfremde Stoffe, bzw. gegen körpereigenes Substrat, daß wegen Fehlregulationen nicht ordnungsgemäß abgebaut wird.
Praktische Anwendung finden:

1. Inaktivierte Viruspräparate (Tierärztl. Praxis 14, 237 - 244, 1986)
Dazu werden Zellkulturen infiziert, nach der Virusvermehrung das Rohvirus mit allen Zellbestandteilen geerntet und anschließend mit physikalischen oder chemischen Methoden inaktiviert (OS 3816139). Das verabreichte Präparat induziert auch eine Immunreaktion
2. Multipotente Paramunitätsmediatoren (DE 3504940)
Es werden Hybridoma-Zellklone verwendet, die mit einem Paramunitätsinducer stimuliert werden müssen. Nach einer entsprechenden Bildungszeit der Mediatoren werden die Zellen lysiert. Angewendet wird ein Stoffgemisch mit relativen Molmassen zwischen 10.000 und 100.000.
Der Nachteil dieser Mediatoren besteht zum einem in der Verwendung der Hybridomzellen und zum anderen in der Verwendung von sogenannten Paramunitätsinducern. Hybridomzellen enthalten immer fremde Erbinformationen, in der Regel Retroviren, die das unbegrenzte Wachstum der Hybridomzellen bedingen. Die Induktion verläuft mit Paramunitätsinducern, die Viren sind. Ein Restrisiko, daß vermehrungsfähige Viren in den zu behandelnden Organismus gelangen, ist nicht auszuschließen.
3. Partikuläre Biokomplexe (G 9218127.9)
Aus Zellkulturen werden in einem aufwendigen Verfahren Partikel mit einem Durchmesser von 50 nm und 9 nm isoliert, die dann in wässeriger Lösung verabreicht werden.
4. Lysate aus menschlichen Leukozyten (Andrologie 9, 379 - 387, 1985)
Menschliche Leukozyten werden lysiert, und mittels Dialyse wird ein Gemisch niedermolekularer Stoffe gewonnen. Viele der "unspezifisch" wirkenden Mediatoren sind als Cytokine nachgewiesen worden. Z.T. wurden ihre Gen-Sequenzen kloniert und ihre Proteinstruktur aufgeklärt. Durch ihre Produktion in Pro- oder Eukaryozyten kann ihr biologischer Effekt in vitro und in vivo nachgewiesen werden. Die Verabreichung einzelner Cytokine bringt häufig nicht den erwarteten therapeutischen Effekt bzw. erst bei Dosierungen, die starke Nebenwirkungen hervorrufen.

Der Erfindung liegt die Aufgabe zugrunde, ein multifaktorielles Abwehr-Modulatorengemisch, unter Nutzung somatischer Zellen als Produzent von Stoffen bereitzustellen, die dazu geeignet sind, am Tier oder Menschen metaphylaktisch oder therapeutisch eingesetzt zu werden. Die somatischen Zellen sollen mit standardisierten Zellinien unter Ausschluß infektiöser oder toxischer Agentien sicher produziert werden, möglichst nicht immunogen sein und Stoffe enthalten, die die Abwehrleistung in ihrer Komplexität unterstützen. Das Abwehr-Modulatorengemisch soll eine erhöhte Wirksamkeit besitzen und geringe Nebenwirkungen aufweisen.

Die Lösung dieser Aufgaben wird gemäß der Patentansprüche 1, 5 und 14 realisiert, die Unteransprüche 2-4, 6-13 und 15 sind Vorzugsvarianten.

Überraschend wurde festgestellt, daß ein solches multifaktorielles Abwehr-Modulatorengemisch mit antiinfektiöser Wirkung gegeben ist durch ein Gemisch von Modulatoren mit einer breiten Molekulargewichtsverteilung, nämlich einer relativen Molmasse > 10.000 und partikulären Bestandteilen < 9 nm.

Bei den wirksamen Modulatoren handelt es sich vorzugsweise um Mediatoren und Wachstumsfaktoren, die ins Medium abgegeben werden wie sauren Fibroblastenwachstumsfaktor, basischen Fibroblastenwachstumsfaktor, Granulocyten-Makrophage Kolonie-stimulierenden Faktor, um zellgebundene Moleküle mit mehr oder minder bekannter Funktion in der Abwehr extrazellulärer Stressoren wie z.B. Ribonukleoproteine und Hitzeschockproteine und um Nukleinsäuren und nukleäre wie cytoplasmatische Proteine wie z.B. Histone, Zellmatrixproteine. Die metaphylaktische und therapeutische Wirkung beruht auf diesem multifaktoriellen Stoffgemisch.

Das erfindungsgemäße Modulatorengemisch enthält vor allem phylogenetisch konservierte Proteine, die nach der durchgeführten Reinigung weitgehend frei von immunogenen Bestandteilen sind. Eine störende Immunreaktion tritt auch bei wiederholter therapeutischer Anwendung nicht auf.

Das Verfahren zur Herstellung dieses multifaktoriellen (multipotenten) Abwehr-Modulatorengemisches erfolgt in Kulturen somatischer Zellen verschiedener Herkunft, wie z.B. Kälbernierenzellinie MDBK, Hundenierenzellinie MDCK, Mausfibroblasten-Zellinie L 929 oder humane Lungenfibroblasten-Zellinien MRC 5 oder MRC 9.

Die Herstellung dieses multipotenten Modulatorengemisches wird durch folgenden Ablauf beschrieben:

1. Kultivierung von Produzentenzellen im Monolayer- oder Suspensionskultur

2. Erhaltung der Zellkulturen unter serumfreien Medien zur Anreicherung von Mediatoren

3. Gewinnung des Zell- und Modulatoren-haltigen Erhaltungsmediums

4. Gewinnung eines teilgereinigten Modulatorengemisches durch Filtration und Ultrafiltration

5. Lagerung des Modulatorengemisches bei bevorzugt 4 °C, -20 °C bzw. lyophilisiert

6. Nachweis der Reinheit, Unschädlichkeit und Wirksamkeit

1. Kultivierung von Produzentenzellen im Monolayer- oder Suspensionskultur

Für die Produktion des Modulatorengemisches werden somatische Zellen unterschiedlichster Herkunft (Insektenzellen, Fischzellen, Reptilienzellen, Warmblüterzellen) eingesetzt, vorzugsweise gut charakterisierte, diploide, begrenzt wachsende Zellinien, die in enger Verwandtschaft zur Zielspezies stehen. Die Produzentenzellen werden in an sich bekannter Weise in Monolayer- oder Suspensionskultur gezüchtet, vorzugsweise als Suspensionskulturen in Spinnergefäßen oder im Fermentor, unter Verwendung üblicher Zellkulturmedien, beispielsweise MEM (minimal essential medium) mit 2 - 8 % Neugeborenen-Kälberserum und 1,5 % NaHCO3. Alle verwendeten Zellkulturmedien enthalten die üblichen Antibiotikazusätze. Die Splitting-Rate ist abhängig von der Vermehrungsgeschwindigkeit der Zellen.

2. Erhaltung der Zellkulturen unter serumfreien Medien zur Anreicherung von Modulatoren

Ist der Monolayer nach 2 - 4 Tagen etwa zu 80 % geschlossen, bzw. hat die Zelldichte in Suspensionskultur etwa 106 Zellen / ml erreicht, wird das serumhaltige Medium ersetzt durch serumfreies MEM. Die Kulturen werden für weitere 3 - 7 Tage bei der zellspezifischen Temperatur (20 bis 39°C) inkubiert. In dieser Zeit schließt sich der Monolayer vollständig, bzw. steigt die Zellzahl in der Suspensionskultur noch leicht an. Mit zunehmender Inkubationszeit verarmt der intrazelluläre Pool an essentiellen Nährstoffen, gleichzeitig werden im MEM Mediatoren und niedermolekulare Stoffwechselendprodukte angereichert. Die Akkumulation von Stoffwechselendprodukten und der intrazelluläre Mangel an essentiellen Nährstoffen stellt letztendlich für die alternde Zellkultur einen Stressfaktor dar auf den sie in phylogenetisch alter Weise durch die Synthese verschiedener "Stress-Proteine" reagiert.

3. Gewinnung des Zell- und Modulatoren-haltigen Erhaltungsmediums

Während der Anreicherungsphase bleiben die Zellen morphologisch intakt. Kulturen mit zytopathischen Veränderungen werden ausgesondert. Nach einer mikroskopischen Kontrolle wird der Monolayer durch Alkalisierung des Erhaltungsmediums mit NaOH auf etwa pH 10 bei gleichzeitiger Temperaturerhöhung auf etwa 50 °C zerstört. Die erfindungsgemäßen Abwehrmodulatoren befinden sich jetzt im Medium. Alle weiteren Arbeiten werden bei Raumtemperatur durchgeführt.

4. Gewinnung eines teilgereinigten Modulatorengemisches durch Zentrifugation oder Filtration und Ultrafiltration

Durch Stufenfiltration werden Zelldetritus und korpuskuläre Bestandteile, die größer als 9 nm sind, abgetrennt. Jetzt steht ein Präparat zur Verfügung, das, von wasserunlöslichen zellulären Membranbestandteilen weitgehend befreit, alle zellgebundenen und extrazellulären Stoffe enthält, die als Modulatorengemisch im Patienten seine biologische Wirksamkeit entfalten. Zur Aufkonzentrierung der wirksamen Bestandteile erfolgt eine Ultrafiltration mit handelsüblichen Geräten (Ausschluß-relative Molmasse < 10.000). Das Retentat enthält das Modulatorengemisch der Erfindung in konzentrierter Form, da eine Volumeneinengung auf das 10 - 20 fache des Ausgangsvolumens erfolgt ist. Eine Abtrennung des Modulatorengemisches kann auch mit anderen Trennverfahren erfolgen.

5. Lagerung des Modulatorengemisches bei 4 °C, -20 °C oder lyophilisiert

Das gewonnene Modulatorengemisch kann bei 4 °C oder -20 °C über Monate ohne Aktivitätsverlust gelagert werden. Es kann auch in bekannter Weise ohne Wirksamkeitsverlust lyophilisiert werden.

6. Nachweis der Reinheit, Unschädlichkeit und Wirksamkeit

Es werden folgende Kontrolluntersuchungen durchgeführt:

Reinheit

1. Prüfung auf Sterilität gemäß Europäischer Pharmakopoe
2. Bestimmung des Proteingehaltes in bekannter Weise
3. Auftrennung und Quantifizierung der Hauptproteine mittels Polyacryamid-Gelelektrophorese

Unschädlichkeit

1. Prüfung auf Pyrogenfreiheit gemäß Europäischer Pharmakopoe / DAB 10
2. Prüfung auf akute Toxizität gemäß Europäischer Pharmakopoe / DAB 10
3. Prüfung auf Teratogenität gemäß Europäischer Pharmakopoe / DAB 10

Wirksamkeit
Zum Nachweis der Wirksamkeit wird multifaktorielles Abwehr-Modulatorengemisch nach den vorstehend beschriebenen Methoden untersucht.

**In-vitro-System**

1. Lymphozytentransformationstest

Die Lymphozytentransformation ist eine entscheidende Frühreaktion in der Organisation der Körperabwehr.
Unter Verwendung von 3H-Thymidin, das in die DNA sich vermehrender Zellen eingebaut wird, kann nachgewiesen werde, daß bestimmte Lymphozyten nach der Zugabe des Mediatorengemisches zur Transformation angeregt werden. Dazu wurden aus frisch gewonnene Heparinblut von Kälbern (homologes System bei Verwendung von MDBK-Zellen), Kaninchen und Menschen (heterologe Systeme bei Verwendung von MDBK-Zellen) die Lymphozyten gewonnen. Etwa 106 Lymphozyten / ml werden zusammen mit 10 µg Modulatorengemisch (gewonnen aus diploider Kälbernieren-Zellinie MDBK (ATCC-Nr. CCL 22)) bzw.2 µg Phythämagglutinin (Positivkontrolle) oder 10 µl MEM (Negativkontrolle) für 7 Tage in RPMI-Medium bei 37 °C inkubiert. Nach dieser Zeit wird den Zellsystemen 3H-Thymidin (etwa 0,2 µCi/ml) zugegeben und nach 5 Stunden weiterer Inkubation werden die Zellen lysiert, das nicht eingebaute 3H-Thymidin ausgewaschen und die spezifische, DNA gebundene Radioaktivität in "counts per minute" (cpm) gemessen und der Stimulationsindex (SI) ermittelt (cpmstimuliert : cpm Kontrolle). Nachfolgend sind typische Ergebnisse zusammengestellt:

| Spezies | Modulatorengemisch cpm | Phythämagglutinin cpm | MEM cpm |
|---------|------------------------|------------------------|---------|
| Rind | 1.120<br>SI 8,40 | 8.930 | 238 |
| Kaninchen | 1.530<br>SI 4,43 | 5.570 | 345 |
| Mensch | 1.020<br>SI 3,80 | 4.780 | 268 |

Die Lymphozyten aller 3 geprüften Spezies reagierten mit einer deutlichen Stimulierung auf die Inkubation mit dem Modulatorengemisch.

2. Phagozytose-Test

Der relative Anteil phagozytierender Granulozyten (= Phagozytosefaktor) und die Anzahl phagozytierter Staphylokokken pro phagozytierender Zelle (Opsoninfaktor) ist ein Maß für die Fähigkeit des Organismus zur unspezifischen Abwehr.

Je 10 frisch gewonnene Proben von sterilen venösen Heparinblut von Kälbern, Kaninchen und Menschen wurde für 60 min mit Modulatorengemisch (gewonnen aus diploider Kälbernieren-Zellinie MDBK (ATCC-Nr. CCL 22)) bzw. MEM (Negativkontrolle) bei 37 °C inkubiert. Anschließend wurden 108 hitzeinaktivierte Staphylokokken dem Blut zugesetzt und für weitere 10 min bei 37 °C inkubiert. In gefärbten Blutausstrichen wird der relative Anteil phagozytierender Granulozyten (= Phagozytosefaktor) und die Anzahl phagozytierter Staphylokokken pro phagozytierender Zelle (Opsoninfaktor) bestimmt. Nachfolgend sind typische Ergebnisse zusammengestellt.

| | Kalb | | Kaninchen | | Mensch | |
|---|---|---|---|---|---|---|
| | MW | SD | MW | SD | MW | SD |
| MEM | | | | | | |
| Phagozytosefaktor | 41,4 | 12,3 | 33,7 | 16,3 | 45,7 | 15,8 |
| Opsoninfaktor | 3,12 | 0,98 | 3,87 | 1,95 | 3,10 | 1,10 |
| Modulatorengemisch | | | | | | |
| Phagozytosefaktor | 67,6 | 9,1 | 59,9 | 10,7 | 66,4 | 9,4 |
| Opsoninfaktor | 3,88 | 1,24 | 4,55 | 1,55 | 3,90 | 1,20 |

Die Phagozytose der Granulozyten im peripheren Blut wird bei allen 3 Spezies unter Einfluß des Modulatorengemisches deutlich stimuliert.

**In-vivo-System**

Infektionsbelastungsversuch mit Maus-adaptierten Newcastle Disease Virus (NCDV) an der Babymaus.

30 BalbC-Babymäuse aus verschiedenen Würfen werden 0,1 ml des Modulatorengemisches verabreicht. Nach 16- 24 Stunden erfolgt eine intraperitoneale Infektion mit etwa 20 LD50 des Maus-adaptierten NCDV. Die Infektion führt nach 4 - 7 Tagen bei etwa 80 % der nichtvorbehandelten Kontrolltiere zum Tod. Als Placebo wird MEM verwendet. Nach 14 Tage wird der Versuch beendet und zur Bewertung der Wirksamkeit der Wirkungsindex für ein Modulatorengemisch (gewonnen aus diploider Kälbernieren-Zellinie MDBK (ATCC-Nr. CCL 22)) mit folgender Formel errechnet.

$$WI = \frac{b - a}{b} \times 100 \qquad b = \text{Mortalität der Kontrollgruppe}$$

$$a = \text{Mortalität der Versuchsgruppe}$$

Ein Wirkungsindex von größer als 20 ist signifikant

| Präparat | Mortalität | WI |
|---|---|---|
| Modulatorengemisch | 10 | 87 |
| MEM | 80 | -- |

Die erfindungsgemäßen Arzneimittel, die über eine wirksame Menge des Modulatorengemisches verfügen und ggf. Zusätze von an sich pharmazeutischen üblichen Trägerstoffen enthalten, sind zur Erhöhung der unspezifischen Abwehr bei Vertebraten geeignet. Diese Arzneimittel dienen zur Metaphylaxe und Therapie von Infektionen verschiedenster Art, speziell von Virusinfektionen, Chemotherapie-resistenten bakteriellen Infek-tionen, von infektiösen Komplexkrankheiten, Mischinfektionen, infektiösen Faktorenkrankheiten, infektiösem Hospitalismus, von hartnäckig rezidivierenden sowie chronischen Infektionen, von stressbedingten Abwehrschwächen und ihren Folgen, von erworbenen (physikalisch, chemisch oder mikrobiell bedingten) Immunsuppressionen und deren Folgen, von altersabhängigen immunregulativen Ausfallerscheinungen, von Autoimmunkrankheiten und immunpathologischen Reaktionslagen, zur Substitutions-therapie bei Tumorerkrankungen und zur Unterstützung einer Chemotherapie bzw. Immunprophylaxe zur Bekämpfung neonataler Krankheiten, die durch einen ungenügenden maternalen Schutz gegenüber der keimhaltigen Umwelt bedingt sind.

Das erfindungsgemäße Abwehr-Modulatorengemisch besitzt folgende Vorteile:

1. Es werden nur standardisierte Zellkulturen und Zellinien verwendet, die frei sind von Fremdviren, eine Induktion mit ehemals infektiösem Material wird vermieden. Dadurch muß keine Beschränkung auf eine subpartikuläre Fraktion mit einem Molekulargewicht zwischen 10.000 und 100.000 wie in DE 3504940 erfolgen, partikuläre Zellbestandteile mit einer Molmasse > 100.000 werden mit in das Gemisch einbezogen.

2. Schnelles und direktes Stimulieren der Effektorzellen durch die Verabreichung eines Modulatoren-Gemisches anstatt deren Induktion. Dadurch können inaktive oder geschädigte Induktorenzellen umgangen und ein eventuelles Mediatorendefizit ausgeglichen werden.

3. Das Modulatorengemisch läßt sich leicht reinigen und anreichern. Unnötige "Ballastproteine" der Zellmembranen werden weitgehend entfernt. Durch die Verwendung isogener Zellinien wird die Antigenität weiter reduziert. Es werden üblicherweise Zellen verwendet, die genau charakterisiert und frei von Viruskontaminanten sind. Die Modulatoren werden ohne Verwendung von infektiösen oder toxischen Stoffen hergestellt.

4. Die Verabreichung des Modulatorengemisches erhöht die Sicherheit einer Prophylaxe oder Therapie, bzw. kann in bestimmten Fällen andere Therapieformen ersetzen.

5. Wenngleich die wirksamen Bestandteile nur teilweise proteinchemisch chrakterisiert sind (Cytokine, bestimmte Stressproteine), lassen die Art der Gewinnung, das Vorhandensein von "Leitsubstanzen" und der in-vitro-Wirksamkeitsnachweis eine Standardisierung zu. Dadurch werden Qualiätsunterschiede, wie sie bei der Herstellung von Proteinlysaten oder Präparaten auf der Grundlage von Blutzellen oder Mikroorganismen entstehen können, verhindert.

6. Durch die Standardisierbarkeit aller Einzelbestandteile läßt sich eine sichere, störungsfreie und kostengünstige Produktion organisieren.

Das erfindungsgemäße Modulatorengemisch bewirkt die konzertierte Reaktion von verschiedenen Abwehrzellen auf vorhandene oder kurzfristig zu erwartende schädigende Einflüsse durch Mikroorganismen, immunpathologische Fehlleistungen oder unkontrollierter Zellproliferation.

Anschließend wird die Erfindung an Ausführungsbeispielen näher erläutert, die die Erfindung jedoch nicht beschränken sollen.

Ausführungsbeispiele

Beispiel 1

Die diploide Kälbernieren-Zellinie MDBK (ATCC-No. CCL 22) wird entsprechend ihrer Vermehrungsrate 1 : 4 in DMEM mit 8 % Neugeborenen-Kälberserum verbracht und in Rollerflaschen bei 38 °C inkubiert. Größere Mengen werden nach Anpassung der Zellen günstigerweise in Spinnerkulturgefäßen oder in Fermentoren angezüchtet. Nach etwa 3 - 4 Tagen ist die Kultur optimal ausgewachsen. Das Anzüchtungsmedium wird entfernt und durch MEM ohne Serum ersetzt.

Die Zellkulturen werden für weitere 5 - 7 Tage inkubiert. Mit zunehmender Inkubationszeit verarmt der intrazelluläre Pool an essentiellen Nährstoffen, gleichzeitig werden im MEM Mediatoren und niedermolekulare Stoffwechselendprodukte angereichert. Die Akkumulation von Stoffwechselprodukten und der intrazelluläre Mangel an essentiellen Nährstoffen stellt letztendlich für die alternde Zellkultur einen Stressfaktor da, auf den sie mit der Synthese verschiedener "Stress-Proteine" reagiert. Diese werden in der Zelle angereichert. Die Zellkulturen werden während der Anreicherungsphase regelmäßig lichtmikroskopisch untersucht. Nach Ablauf der Anreicherungsphase dürfen keine zytopathischen Veränderungen sichtbar sein. Die Anreicherungsphase wird mit der Alkalisierung des MEM mit NaOH auf pH 10 und der Temperaturerhöhung auf 50 °C für 60 min beendet.

Statt der pH-Wert Verschiebung hat sich auch das Abfrieren der Zellen bewährt. Das geschieht günstigerweise bei -20 °C. Nach dieser Zeit können die abgestorbenen Zellen ohne Mühe von der Kulturflaschenoberfläche abgeschüttelt werden.

Temperaturerhöhung und pH-Shift haben keinen Einfluß auf die biologische Aktivität des Modulatorengemisches.

Nun wird das Erhaltungsmedium mit den extra- und intrazellulären Modulatoren durch Stufenfiltration (< 9 nm) von Zelldetritus und partikulären Bestandteilen befreit. Das gewonnene Filtrat enthält das Gemisch von Modulatoren, weitgehend befreit von Membranbestandteilen. Es kann steril ohne Aktivitätsverlust für mehrere Wochen bei 4 °C aufbewahrt werden bzw. eingefroren (- 20 °C und tiefer) für mindestens ein Jahr.

Zur Gewinnung eines angereicherten Modulatorengemisches wird eine Ultrafiltration mit handelüblichen Geräten vorgenommen. Die Porengröße der Filtrationsmembranen halten globuläre Moleküle ab einer relativen Molmasse von etwa 10.000 zurück. Im Retentat befinden sich alle extra- und intrazellulären Stoffe mit einer relativen Molmasse für globuläre Proteine von mehr als 10.000 und Partikel mit einen Durchmesser von weniger als 9 nm.

Das so angereicherte Modulatorengemisch kann steril ohne Aktivitätsverlust für mehrere Wochen bei 4 °C aufbewahrt werden bzw. eingefroren (- 20 °C und tiefer) für mindestens ein Jahr. Es kann auch in üblicher Weise lyophilisiert werden. Die Reinheit, Unschädlichkeit und Wirksamkeit des erfindungsgemäßen Modulatorengemisches wird durch entsprechende Kontrolluntersuchungen überprüft. Die Reinheits- und Unschädlichkeitsprüfungen erfolgen nach den vorgeschriebenen Methoden der Europäischen Pharmakopoe bzw. des DAB 10. Proteinbestimmung und Polyacrylgel-Elektrophorese werden nach altbekannten Standardprotokollen durchgeführt.

Reinheit

    1. Prüfung auf Sterilität gemäß Europäischer Pharmakopoe / DAB 10
    2. Bestimmung des Proteingehaltes nach Lowry
    3. Auftrennung und Quantifizierung der Hauptproteine mittels Polyacryamid-Gelelektrophorese unter denaturierenden Bedingungen.

Unschädlichkeit

    1. Prüfung auf Pyrogenfreiheit gemäß Europäischer Pharmakopoe / DAB 10
    2. Prüfung auf akute Toxizität gemäß Europäischer Pharmakopoe / DAB 10
    3. Prüfung auf Teratogenität gemäß Europäischer Pharmakopoe / DAB 10

Eine typische Präparation ist steril, pyrogenfrei, nicht toxisch und nicht teratogen. Es entält etwa 2 - 3 mg Protein pro ml.

Beispiel 2

Hühnerfibroblasten, gewonnen durch tryptische Zellvereinzelung von Hühnerembryonen, werden gemäß Beispiel 1 in Rollerflaschen kultiviert. Anreicherung, Ernte und Aufbereitung sowie Prüfung auf Identität, Unschädlichkeit und Wirksamkeit erfolgte gemäß Beispiel 1.

Es werden im wesentlichen die gleichen Ergebnisse wie in Beispiel 1 erzielt.

Beispiel 3

Eine diploide, begrenzt wachsende Tubenepithel-Zellinie vom Schwein (Latzke,1993) wurde gemäß Beispiel 1 stationär kultiviert. Anreicherung, Ernte und Aufbereitung sowie Prüfung auf Identität, Unschädlichkeit und Wirksamkeit erfolgte gemäß Beispiel 1.

Es werden im wesentlichen die gleichen Ergebnisse wie in Beispiel 1 erzielt.

Beispiel 4

Gemäß Beispiel 1 - 3 hergestellte Zellkulturen werden mit Beginn der Anreicherungsphase zusätzlich mit dem Modulatorengemisch (1 mg/l MEM) inkubiert. Die Induktion mit dem Modulatorengemisch ermöglichte eine frühere Ernte. Aufbereitung sowie Prüfung auf Identität, Unschädlichkeit und Wirksamkeit erfolgte gemäß Beispiel 1.
Es werden im wesentlichen die gleichen Ergebnisse wie in Beispiel 1 erzielt.

Beispiel 5

Gemäß Beispiel 1 - 4 hergestellte Modulatorengemische sind bei Lagerung in flüssiger Form bei 4 °C über Wochen stabil oder sie werden lyophilisiert. In dieser Zubereitung sind sie sofort bzw. nach Resuspension in Aqua ad injectionem für die parenterale oder lokale Verabreichung anwendbar.
Das erfindungsgemäße Modulatorengemisch kann lyophilisiert oder flüssig auch in andere pharmazeutische Träger eingearbeitet werden.
Die Dosierung, Applikationsort und Applikationshäufigkeit richtet sich nach der Art der Erkrankung. Dabei gibt es keinen prinzipiellen Unterschiede zwischen einer Anwendung in der Humanmedizin und der Veterinärmedizin. Chronische Erkrankungen verlangen eine sich in längeren Abständen wiederholende Anwendung über jeweils mehrere Tage. Bei akuten Infektionen werden dagegen beispielsweise 1 - 3 mal täglich über 3 - 5 Tage je 1 - 2 ml intramuskulär verabreicht. Da keine Gefahr der Überdosierung besteht, wird man in der Humanmedizin 2 - 3 ml verabreichen. In der Veterinärmedizin wird man üblicherweise Kleintiere mit 0,5 - 1 ml behandeln, während bei Großtiere 4 - 5 ml Modulatorengemisch angebracht erscheinen.

Beispiel 6

Die erfindungsgemäße Anwendung des Modulatorengemisches in der Prophylaxe und Therapie von unterschiedlichen Krankheiten wird durch einige Beispiele aus der Human- und Veterinärmedizin belegt.

1. Metaphylaxe und Therapie von Pneumonien in der Kälberaufzucht

Da eine Nichtbehandlung erkrankter Tiere aus betriebswirtschaftlichen Gründen nicht möglich ist, wurde der Einsatz des Modulatorengemisches verglichen mit dem Effekt der üblichen antibiotischen Behandlung. Insgesamt wurden 103 Kälbern zu Beginn der Erkrankung je 1 ml des Mediatorengemisches intramuskulär verabreicht. Bei klinischer Notwendigkeit wurde in gleicher Dosierung nachbehandelt. Verglichen wurden Morbidität am ersten Tag nach der Behandlung, Rezidive und notwendige Behandlungen
Folgende Ergebnisse wurden erzielt:

|  | Mediatorengemisch | | Antibiotika-Behandlung | |
|---|---|---|---|---|
|  | absolut | relativ | absolut | relativ |
| Anzahl von Tieren | 103 |  | 305 |  |
| Morbidität | 52 | 52 % | 188 | 62% |
| Rezidive | 33 | 34 % | 133 | 44 % |
| Behandlungen | 323 |  | 1187 |  |
| Behandlungen/Kalb | 3,13 |  | 3,89 |  |
| Behandlungen pro 100 Tiere | 313 |  | 390 |  |

Es zeigt sich, daß das Modulatorengemisch einen deutlichen therapeutischen Effekt ausübt, der in der erprobten Anwendung der Wirkung von herkömmlichen Antibiotika entspricht.

2. Therapie von Erkrankungen der oberen Atemwege des Menschen

18 erwachsene Freiwillige setzten mit Beginn der Erkältungserkrankung das Mediatorengemisch in Form von Nasentropfen und als Inhalationsmittel ein. Bereits nach 24 Stunden waren alle Symptome abgeklungen.

3. Behandlung von Katzenschnupfen

Insgesamt wurden 53 Katzen einer Versuchstierhaltung einmalig mit 1ml Mediatorengemisch intramuskulär behandelt. Bereits nach 24 Stunden waren die Symptome abgeklungen, während nicht behandelte Tiere aus dem gleichen Bestand eine unveränderte Klinik zeigten.

4. Behandlung von Keratokonjunktivitiden bei Katzen

Weitgehend therapieresistente Keratokonjunktivitiden bei 43 Jungtieren (Alter 4 - 8 Wochen) in einer Versuchstierhaltung konnten durch einmalige parenterale Anwendung des Mediatorengemisches kombiniert mit einer dreitägigen lokalen Anwendung als Augentropfen erfolgreich behandelt werden.
Bei konventionell behandelten Tieren konnten trotz hohen Medikamenten- und Pflegeaufwandes schwere Verlaufsformen, die zur Einschränkung der Sehfähigkeit führten, nicht völlig vermieden werden.

5. Prophylaxe des infektiösen Welpensterbens

4 Mutterhündinnen wurden in der letzten Trächtigkeitwoche mit je 1 ml Mediatorengemisch parenteral behandelt. Die Welpen erhielten sofort nach der Geburt 0,5 ml intramuskulär und eine 2. Applikation nach 24 Stunden verabreicht.
3 Mutterhündinnen und ihre Würfe dienten als Kontrolle.
Die behandelten Tiere warfen 11 Welpen, die alle aufgezogen werden konnten, von den 8 geworfenen Welpen der Kontrolltiere konnten nur 4 aufgezogen werden, 4 verendeten innerhalb der ersten 2 Lebenswochen.
Die ausgeführten Beispiele beweisen die Wirksamkeit des erfindungsgemäßen Modulatorengemisches an Mensch und Tier. Die Zubereitungen sind unschädlich. Nebenreaktionen wurden nicht festgestellt.

**Patentansprüche**

**1.**　Multifaktorielles Abwehr-Modulatorengemisch mit antiinfektiöser Wirkung bestehend aus extrazellulären gelösten und intrazellulären Proteinen mit einer relativen Molmasse von mehr als 10.000 und partikulären Bestandteilen mit einem Durchmesser kleiner als 9 nm und gewonnen aus somatischen Zellen

- durch Inkubation bei den für die Ursprungsspezies typischen Temperaturen von 20 bis 39 °C über einen Zeitraum von 2 - 8 Tagen,
- anschließender Lyse,
- Ernte zusammen mit dem Erhaltungsmedium und
- Abtrennung von Zelltrümmern, die größer als 9 nm sind, und Partikeln mit einer Molmasse < 10.000 durch Filtration und Ultrafiltration mit den Eigenschaften

　　a) Vermittlung einer erhöhten Phagozytoseleistung von phagozytierenden Zellen und einer erhöhten Stimulierbarkeit von Lymphozyten und
　　b) Vermittlung eines Infektionsschutzes der Maus gegen eine experimentelle Infektion mit einem Mausadaptierten NCDV-Stamm.

**2.**　Multifaktorielles Abwehr-Modulatorengemisch nach Anspruch 1, dadurch gekennzeichnet, daß es aus primären Zellkulturen oder aus begrenzt wachsenden diploiden oder unbegrenzt wachsenden polyploiden Zellinien gewonnen wird.

**3.**　Multifaktorielles Abwehr-Modulatorengemisch nach Anspruch 1 und 2, dadurch gekennzeichnet, daß es sich aus Mediatoren und Wachstumsfaktoren zusammensetzt.

**4.**　Multifaktorielles Abwehr-Modulatorengemisch nach Anspruch 3, dadurch gekennzeichnet, daß die Mediatoren und Wachstumsfaktoren Acidic Fibroblast Growth Factor, Basic Fibroblast Growth Factor, Granulocyte-Makrophage Colony-Stimulating Factor, zellgebundene Moleküle mit der Funktion der Abwehr extrazellulärer Stressoren, Nukleinsäuren, nukleäre Proteine und Zellmatrixproteine sind.

**5.**　Verfahren zur Herstellung eines multifaktoriellen Abwehr-Modulatorengemisches gemäß der Ansprüche 1 - 4 durch Inkubation von Zellkulturen somatischer Zellen unterschiedlichster Herkunft bei den für die Ursprungsspezies typischen Temperaturen über einen Zeitraum von 2 - 8 Tagen, ggf. zur Beschleunigung der Anreicherung Belastung mit chemischen, physikalischen oder biologischen Mitteln, anschließender Lyse, Ernte zusammen mit dem Erhaltungsmedium, Abtrennen des Zelldetritus >9nm durch Filtration, Ultrafiltration zum Aufkonzentrieren der wirksamen Bestandteile und zum Ausschluß der Substanzen mit einer Molmasse <10.000 und ggf. anschließender Lyophilisierung.

6. Verfahren nach Anspruch 5 durch Inkubation von primären Zellkulturen oder aus begrenzt wachsenden diploiden oder unbegrenzt wachsenden polyploiden Zellinien.

7. Verfahren nach Anspruch 5 und 6 durch Inkubation der diploiden Kälbernieren-Zellinie MDBK, ATCC-No. CCL 22.

8. Verfahren nach Anspruch 5 und 6 durch Inkubation von Hundenierenzellinie MDCK, ATCC-No. CCL 34.

9. Verfahren nach Anspruch 5 und 6 durch Inkubation von Mausfibroblasten-Zellinie L 929, ATCC-No. CCL 1.

10. Verfahren nach Anspruch 5 und 6 durch Inkubation von humanen Lungenfibroblasten-Zellinien MRC 5 und MRC 9, ATCC-No. CCL 171 und 212.

11. Verfahren nach Anspruch 5 und 6 durch Inkubation von Hühnerfibroblasten.

12. Verfahren nach Anspruch 5 und 6 durch Inkubation einer diploid, begrenzt wachsenden Tubenepithel-Zellinie vom Schwein.

13. Verfahren nach Anspruch 5 bis 12 durch Zusatz von multifaktoriellem Abwehr-Modulatorengemisch mit Beginn der Anreicherungsphase zur Beschleunigung der Anreicherung.

14. Arzneimittel zur Metaphylaxe und Therapie von Infektionen verschiedenster Art zur Erhöhung der unspezifischen (nicht erregerspezifischen) Abwehr bei Vertebraten enthaltend eine wirksame Menge des multifaktoriellen Abwehr-Modulatorengemisches gemäß der Ansprüche 1 -4 und ggf. Zusätze von an sich üblichen pharmazeutischen Trägern für die parenterale oder lokale Verabreichung.

15. Arzneimittel nach Anspruch 14 zur Metaphylaxe und Therapie von Virusinfektionen, Chemotherapie-resistenten bakteriellen Infektionen, von infektiösen Komplexkrankheiten, Mischinfektionen, infektiösen Faktorenkrankheiten, infektiösen Hospitalismus, von hartnäckig rezivierenden sowie chronischen Infektionen, von stressbedingten Abwehrschwächen und ihren Folgen, von erworbenen Immunsuppressionen und deren Folgen, von altersabhängigen immunregulativen Ausfallerscheinungen, von Autoimmunkrankheiten und immunpathologischen Reaktionslagen, zur Substitutionstherapie bei Tumorerkrankungen und zur Unterstützung einer Chemotherapie bzw. Immunprophylaxe zur Bekämpfung neonataler Krankheiten, die durch einen ungenügenden maternalen Schutz gegenüber der keimhaltigen Umwelt bedingt sind.

## Claims

1. Multi-factorial defence modulator mixture with anti-infectious effect, comprising extra-cellular dissolved and intra-cellular proteins with a relative mol mass of more than 10,000 and particular components with a diameter of less than 9 nm, obtained from somatic cells

   - by incubation at the temperatures of 20 to 39 °C typical for the original species for a period of 2-8 days,
   - subsequent lysis,
   - harvesting together with the conservation agent and
   - separation of cell detritus larger than 9 nm and particles with a mol mass of < 10,000 by filtration and ultra-filtration with the properties

     a) production of an increased phagocytosis performance of phagocytising cells and an increased stimulation capacity of lymphocytes and
     b) production of an infection protection of the mouse against an experimental infection with a mouse-adapted NCDV strain.

2. Multi-factorial defence modulator mixture in accordance with claim 1, wherein it is obtained from primary cell cultures or from limited-growth diploid or unlimited-growth polyploid cell lines.

3. Multi-factorial defence modulator mixture in accordance with claims 1 and 2, wherein it is composed of mediators and growth factors.

4. Multi-factorial defence modulator mixture in accordance with claim 3, wherein the mediators and growth factors Acidic Fibroblast Growth Factor, Basic Fibroblast Growth Factor, Granulocyte Macrophage Colony-Stimulating Factor, are cell-bound molecules with the function of defence of extra-cellular stressors, nuclein acids, nuclear proteins and cell matrix proteins.

5. Method for the production of a multi-factorial defence modulator mixture in accordance with claims 1-4 by incubation of cell cultures of somatic cells of various origins at the temperatures typical for the original species for a period of 2-8 days, if need be, to accelerate the enrichment, stressing with chemical, physical or biological agents, subsequent lysis, harvesting together with the conservation agent, separation of the cell detritus <9nm by filtration, ultra-filtration for a concentration of the effective components and to exclude the substances with a mol mass of <10,000 and, if need be, subsequent lyophilisation.

6. Method in accordance with claim 5 by incubation of primary cell cultures or from limited-growth diploid or unlimited-growth polyploid cell lines.

7. Method in accordance with claims 5 and 6 by incubation of the diploid calves' kidney cell line MDBK, ATCC no. CCL 22.

8. Method in accordance with claims 5 and 6 by incubation of dogs' kidney cell line MDCK, ATCC no. CCL 34.

9. Method in accordance with claims 5 and 6 by incubation of mouse fibroblast cell line L 929, ATCC no. CCL 1.

10. Method in accordance with claims 5 and 6 by incubation of human lung fibroblast cell lines MRC 5 and MRC 9, ATCC no. CCL 171 and 212.

11. Method in accordance with claims 5 and 6 by incubation of chicken fibroblasts.

12. Method in accordance with claims 5 and 6 by incubation of a diploid, limited-growth tube epithelium cell line of pigs.

13. Method in accordance with claims 5 to 12 through addition of a multi-factorial defence modulator mixture at the start of the enrichment phase in order to accelerate the enrichment.

14. Medication for the metaphylaxis and therapy of infections of various kinds for increasing the unspecific (not pathogen-specific) defence in vertebrates, containing an effective quantity of the multi-factorial defence modulator mixtures in accordance with claims 1 - 4 and if need be additives of basically customary pharmaceutical carriers for parenteral or local administration.

15. Medication in accordance with claim 14 for the metaphylaxis and therapy of virus infections, chemotherapy-resistant bacterial infections, infectious complex diseases, mixed infections, infectious factor diseases, infectious hospitalism, stubbornly recurring and chronic infections, stress-induced defence weakness and its consequences, acquired immune suppressions and their consequences, age-dependent immuno-regulative loss phenomena, auto-immune diseases and immuno-pathological reaction situations, for substitution therapy in tumour diseases and to support a chemotherapy or immunoprophylaxis to combat neonatal diseases caused by insufficient maternal protection against the environment containing germs.

**Revendications**

1. Mélange de modulateurs de défense multifactoriel à action anti-infectieuse composé de protéines extra-cellulaires dissoutes et de protéines intra-cellulaires d'une masse molaire relative de plus de 10.000 et de particules d'un diamètre inférieur à 9 nm, provenant de cellules somatiques

- par incubation aux températures typiques pour les espèces d'origine de 20 à 39 °C pendant une durée de 2 à 8 jours,
- lyse consécutive,
- collecte avec l'agent de conservation et
- séparation des débris de cellules supérieurs à 9 nm et des particules d'une masse molaire < 10.000 par filtration et ultra-filtration, ayant les propriétés

    a) acquisition d'un pouvoir phagocytaire accru des cellules phagocytes et capacité de stimulation accrue des lymphocytes et
    b) acquisition d'une protection immunologique de la souris contre l'infection expérimentale avec une colonie NCDV adaptée à la souris.

2. Mélange de modulateurs de défense multifactoriel selon la revendication 1, caractérisé en ceci qu'il est obtenu à partir de cultures de cellules primaires ou de lignes de cellules diploïdes à croissance limitée ou de lignes cellulaires polyploïdes à croissance illimitée.

3. Mélange de modulateurs de défense multifactoriel selon les revendications 1 et 2, caractérisé en ceci que qu'il s'agit d'une combinaison de médiateurs et de facteurs de croissance.

4. Mélange de modulateurs de défense multifactoriel selon la revendication 3, caractérisé en ceci que les médiateurs et facteurs de croissance Acidic Fibroblast Growth Factor, Basic Fibroblast Growth Factor, Colony-Stimulating Factor macrophage des granulocytes sont des molécules liées aux cellules ayant pour fonction d'assurer la défense par rapport aux agents d'agression extra-cellulaires, des acides nucléiques, des protéines nucléiques et les protéines de matrice cellulaire.

5. Procédé de fabrication d'un mélange de modulateurs de défense multifactoriel selon les revendications 1 à 4 par incubation de cultures de cellules somatiques d'origine différente aux températures typiques pour les cellules d'origine pendant une durée de 2 à 8 jours, si nécessaire avec accélération de la charge d'enrichissement par les agents chimiques, physiques et biologiques, ensuite lyse, récolte avec l'agent de conservation, séparation du détritus cellulaire >9 nm par filtration, ultra-filtration pour assurer la concentration des principes actifs et rejet des substances d'une masse molaire <10.000 et, si nécessaire, lyophilisation consécutive.

6. Procédé selon la revendication 5 par incubation de cultures primaires de cellules ou de lignes de cellules diploïdes à croissance limitée et de cellules polyploïdes à croissance illimitée.

7. Procédé selon les revendications 5 et 6 par incubation des lignes de cellules rénales de veau diploïdes MDBK, n° ATCC CCL 22.

8. Procédé selon les revendications 5 et 6 par incubation de lignes de cellules rénales de chien MDCK, n° ATCC CCL 34.

9. Procédé selon les revendications 5 et 6 par incubation de lignes de cellules de fibroblaste de souris L 929, n° ATCC CCL 1.

10. Procédé selon les revendications 5 et 6 par incubation de lignes de cellules de fibroblaste pulmonaire humain MRC 5 et MRC 9, n° ATCC CCL 171 et 212.

11. Procédé selon les revendications 5 et 6 par incubation de fibroblastes de poule.

12. Procédé selon les revendications 5 et 6 par incubation d'une ligne de cellule d'hépithéliome tubulé diploïde de porc à croissance limitée.

13. Procédé selon les revendications 5 à 12 par adjonction du mélange de modulateurs de défense multifactoriel au début de la phase d'enrichissement pour accélérer l'enrichissement.

14. Médicament de traitement métaphylaxique et de traitement primaire des infections de diverse nature pour l'accroissement de la défense non spécifique (non spécifique de l'agent pathogène) chez les vertébrés, contenant une quantité efficace du facteur de modulateurs de défense multifactoriel selon les revendications 1 à 4 et si nécessaire avec adjonction des supports pharmaceutiques usuels pour l'application parentérale et locale.

15. Médicament selon la revendication 14 pour le traitement métaphylaxique et le traitement primaire des infections virales, des infections microbiennes résistant à la chimiothérapie, des maladies multiples de nature infectieuse, des infections mixtes, des maladies à facteur infectieux, des infections nosocomiales, des infections récidivantes opiniâtres et chroniques, des faiblesses immunologiques provoquées par le stress, des insuffisances immunitaires acquises et de leurs conséquences, des phénomènes de dégénérescence de la régulation immunitaire dues à l'âge, les maladies autoimmunes et des situations de réactions immunologiques pathologiques, pour le traitement de substitution des maladies cancéreuses et l'assistance de la chimiothérapie et la prévention immunologique pour la lutte contre les maladies néo-natales dues à une protection maternelle insuffisante contre les germes du milieu.